(19) Europäisches Patentamt
European Patent Office
Office européen des brevets

(11) Numéro de publication: **0 057 653**
**B1**

(12) # FASCICULE DE BREVET EUROPEEN

(45) Date de publication du fascicule du brevet: **17.04.85**

(51) Int. Cl.⁴: **C 08 G 18/02**, C 07 D 251/34

(21) Numéro de dépôt: **82420019.0**

(22) Date de dépôt: **01.02.82**

(54) **Procédé d'obtention de composés à groupements isocyanuriques par cyclotrimérisation catalytique d'isocyanates à l'aide de composés à groupement amino silylés-isocyanurates obtenus par la mise en oeuvre du procédé.**

(30) Priorité: **03.02.81 FR 8102192**
**09.12.81 FR 8123135**

(43) Date de publication de la demande:
**11.08.82 Bulletin 82/32**

(45) Mention de la délivrance du brevet:
**17.04.85 Bulletin 85/16**

(84) Etats contractants désignés:
**AT BE CH DE FR GB IT LI LU NL SE**

(56) Documents cités:
**CH - A - 422 781**
**FR - A - 1 434 769**

**JOURNAL OF THE CHEMICAL SOCIETY, Section C, 1969 LETCHWORTH (GB) KENJI ITOH et al.: "Reactions of Group IV Organometallic Compounds. Part XIV. Anomalous cyclization products from the reaction of heptamethyldisilazane with phenyl isocyanate at elevated temperatures" pages 2005-2007**

(73) Titulaire: **RHONE-POULENC SPECIALITES CHIMIQUES, "Les Miroirs" 18, Avenue d'Alsace, F-92400 Courbevoie (FR)**

(72) Inventeur: **Robin, Jean, 21, rue Duguesclin, F-69006 Lyon (FR)**

(74) Mandataire: **Chichery, Guy et al, RHONE-POULENC RECHERCHES Service Brevets Chimie et Polymères Centre de Recherches de Saint-Fons B.P. 62, F-69192 St-Fons Cédex (FR)**

**EP 0 057 653 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne un procédé d'obtention de composés à groupements isocyanuriques par cyclotrimérisation catalytique de mono ou de polyisocyanates, la catalyse étant initiée par mise en contact de l'isocyanate de d'un composé à groupements aminosilylés tel qu'un aminosilane, une silylurée ou un silazane.

On connait de nombreux procédés d'obtention de composés à groupements isocyanuriques simples ou polymériques par réaction de cyclotrimérisation catalytique d'isocyanates aliphatiques, cycloaliphatiques ou aromatiques. La réaction de cyclotrimérisation peut être totale ou partielle. On peut ainsi obtenir à partir de monoisocyanates des monoisocyanurates alors que l'on préparera à partir de polyisocyanates des polyisocyanates polyisocyanurates par cyclotrimérisation partielle, et des polyisocyanurates sans groupement isocyanate libre par cyclotrimérisation totale. Ces derniers constituent des matériaux polymériques très fréquemment utilisés sous forme alvéolaire (mousses polyisocyanuriques).

En ce qui concerne l'obtention d'isocyanurate ou de polyisocyanurate par trimérisation totale de monoisocyanates ou de polyisocyanates aliphatiques, cycloaliphatiques ou aromatiques on citera à titre de catalyseurs les composés suivants: les amines tertiaires, les phosphines, les alcoolates, les dérivés des métaux alcalins ou alcalino-terreux, tels que leurs oxydes, leurs hydroxydes, leurs carboxylates ... Ces catalyseurs sont par exemple décrits dans Journal of Cellular Plastics Janvier 1965 p. 85 à 90; Journal Macromolecular Science Reviews. Macromolecular Chemistry (5/1) 105—109 (1970). Certains dérivés du silicium et de l'étain ont été également décrits comme catalyseurs de la réaction de cyclotrimérisation de phénylisocyanate en isocyanurate. Itoh, Matsuzaki et Ishii ont ainsi montré le rôle catalytique du sulfure de bis triméthylsilyle [Journal Chemical Society (C) 2709—2712 (1968)] et des N-silylamines [(Journal Chemical Society (C) 2005—2007 (1969)]. On notera que les triméthylsilylamines permettent de cyclotrimériser de manière très lente l'isocyanatobenzène en isocyanurate (essais effectués durant 42 heures à 150°C). Les silylamines apparaissent donc être des catalyseurs de cyclotrimérisation très peu actifs pour les isocyanates aromatiques. Par ailleurs les autres précédemment nommés ont également montré que les disilazanes (hexaméthyldisilazane; heptaméthyldisilazane, hexaméthyl, N-éthyldisilazane) n'étaient pas des catalyseurs de cyclotrimérisation du phénylisocyanate en triphénylisocyanurate: ces silazanes se sont avérés être au contraire des réactifs conduisant avec le phénylisocyanate à des imino, triphénylhexahydrotriazines dione [par exemple la méthylimino-4, triphényl-1,3,5 hexahydrotriazine-1,3,5 dione-2,6 et la triphényl-1,3,5 tris(phénylimino)-2,4,6 hexahydro triazine-1,3,5 sont obtenues à partir d'heptaméthyldisilazane].

Les polyisocyanates polyisocyanurates qui sont des constituants de base pour les vernis et les peintures sont obtenus en général par cyclotrimérisation partielle des groupements NCO, des polyisocyanates simples aliphatiques ou aromatiques ou de polyisocyanates adducts à l'aide de catalyseurs variés tels que les amines tertiaires (brevet allemand 951 168), les dérivés des métaux alcalins ou alcalino-terreux tels que hydroxyde, carbonate, alcoolate [brevet français 1 190 065], les hydroxydes d'ammonium quaternaire [brevet français 1 204 697; 1 566 256; demande de brevet européen 0 003 765 et 10 589], les phosphines [brevet français 1 510 342; 2 023 423; demande de brevet allemand 1 934 763], les catalyseurs à groupement éthylèneimine [brevet français 1 401 513; 2 230 642] et enfin les bases de Mannich [brevet français 2 290 459 et 2 332 274]. Ces divers catalyseurs peuvent en outre éventuellement être associés avec un ester carbamique [amine tertiaire + carbamate: brevet français 1 172 576; dérivés de métal alcalin ou alcalino-terreux + carbamate: brevet français 1 304 301; base de Mannich + carbamate: brevet français 2 290 459].

Le catalyseur de cyclotrimérisation partielle des groupements NCO doit de manière générale être désactivé lorsque l'on a atteint la teneur désirée en groupement isocyanate libre. Cette désactivation peut être effectuée par addition de composé acide (hydracide, chlorure d'acide ...), d'agent alkylant (iodure de méthyle...) d'agent acylant. Enfin la désactivation peut être faite par un traitement thermique approprié.

On connaissait ainsi divers systèmes catalytiques qui permettaient de trimériser les isocyanates aliphatiques ou cycloaliphatiques en composés isocyanuriques (isocyanurates simples ou polyisocyanates polyisocyanurates) mais ces divers systèmes présentent tous des inconvénients. La catalyse par les dérivés des métaux alcalins et alcalino-terreux se manifeste toujours de manière imprévisible avec un certain décalage dans le temps puis est soudaine et en général difficile à contrôler du fait de son activité trop importante. Avec des systèmes catalytiques moins réactifs on n'observe plus le retard à l'activité catalytique mais la catalyse est peu efficace. Il est nécessaire de cyclotrimériser à température élevée ce qui provoque la formation d'une quantité non négligeable de dimère. Par ailleurs il faut en général chauffer durant la période de désactivation du catalyseur ce qui favorise également la formation de dimère.

Il se posait donc le problème de trouver un système catalytique qui permette de cyclotrimériser les isocyanates aliphatiques ou cycloaliphatiques en composés à groupements isocyanuriques, la réaction catalytique s'effectuant sans période d'induction et de manière régulière à une température relativement modérée. Il importait par ailleurs qu'un tel catalyseur puisse être aisément désactivé; la présente invention répond précisément à ce but.

Il a été maintenant trouvé et c'est ce qui constitue l'objet de la présente invention, un procédé d'obtention de composés à groupements isocyanuriques monomères ou polymères par cyclotrimérisation

catalytique d'isocyanate dans lesquels le groupement isocyanate n'est pas directement relié à un atome de carbone appartenant à un groupement aromatique, la cyclotrimérisation étant effectuée par mise en contact de l'isocyanate et d'un composé initiant la réaction catalytique, le procédé étant caractérisé en ce que l'on utilise à titre de composé initiant la réaction catalytique un composé à groupement aminosilylé et ayant pour formule (I):

$$R_{(4-n)}Si-[NR'R'']_n \qquad (I)$$

dans laquelle les divers symboles représentent respectivement:

- R: un radical monovalent de nature hydrocarbonée, aliphatique, cycloaliphatique saturé ou insaturé, aryle, aralkyle ou alkylaryle, éventuellement substitué par des atomes d'halogène ou des groupements CN, deux radicaux R pouvant constituer ensemble un radical hydrocarboné divalent,
- R': un radical monovalent choisi parmi les radicaux R, $SiR_3$, ou les radicaux amide de formule:

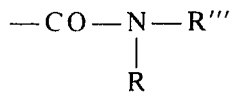

  R''' représentant R ou $SiR_3$, R ayant la signification préalablement donnée, le radical R' pouvant éventuellement lorsqu'il ne représente pas un groupement amide ou un groupement $SiR_3$ constituer avec le radical R'' un radical hydrocarboné divalent,
- R'': un radical monovalent ayant la même signification que le radical R; ou un atome d'hydrogène lorsque R' n'est pas un radical amide
- n: un nombre entier égal à 1 ou 2. Lorsque n est égal à 2, R' est un radical R.

Le composé initiant la réaction de cyclotrimérisation en isocyanurate et qui sera plus commodément désigné par le terme de catalyseur s'est révélé particulièrement intéressant pour la cyclotrimérisation des isocyanates aliphatiques ou cycloaliphatiques. Le catalyseur qui peut être un aminosilane, un diaminosilane, une silylurée ou un silazane est remarquable en ce que son action catalytique est immédiate, régulière et importante à température modérée. Un tel résultat est inattendu. On devait en effet s'attendre au vu de la plus faible réactivité que présentent habituellement les isocyanates aliphatiques vis-à-vis des isocyanates aromatiques [cf par exemple Polyurethanes Chemistry and technology part I chemistry p. 64 par. J. H. SAUNDERS et K. C. FRISCH Interscience publishers (1962)], à obtenir une activité catalytique très atténuée étant donné qu'une très faible activité catalytique est observée avec les isocyanates aromatiques. C'est l'effet contraire qui a été observé.

Par ailleurs lors de l'emploi de silazanes en tant que catalyseurs on obtient comme on l'a déjà dit des isocyanurates en série isocyanates aliphatiques ou cycloaliphatiques. Ce résultat est totalement inattendu puisqu'Itoh et al. ont montré qu'en série isocyanate aromatique, on obtenait des iminohexahydrotriazines.

Plus précisément la présente invention concerne un procédé d'obtention de composés à groupements isocyanuriques par catalyse aux composés à groupement aminosilylés et ayant pour formule (I) dans laquelle les divers symboles réprésentent respectivement:

- R: un radical alkyle, alkényle ou halogénoalkyle ou halogénoalkényle ayant de 1 à 5 atomes de carbone et comportant de 1 à 6 atomes de chlore et/ou de fluor des radicaux cycloalkyle, cycloalkényle et halogénocycloalkyle, halogénocycloalkényle ayant de 3 à 8 atomes de carbone et contenant de 1 à 4 atomes de chlore et/ou de fluor
  des radicaux aryles, alkylaryles et halogénoaryles ayant de 6 à 8 atomes de carbone et contenant de 1 à 4 atomes de chlore et/ou de fluor
  des radicaux cyanoalkyles ayant de 3 à 4 atomes de carbone

- deux symboles R portés par un même atome de silicium constituent entre eux un radical divalent ayant de 1 à 4 atomes de carbone.

- R': un radical monovalent choisi par les radicaux R, $SiR_3$, et CO(NR)—R''', R''' représentant R ou $SiR_3$, R ayant la signification plus précise qui vient juste d'être donnée ci-dessus; R' pouvant constituer avec R'' un radical alkylène ayant de 4 à 6 atomes de carbone.
- R'': un radical alkyle ou alkényle ayant de 1 à 4 atomes de carbone, un radical cycloalkyle ou cycloalkényle ayant de 4 à 6 atomes carbone nucléaires, un radical phényle ou tolyle ou xylyle, ou un atome d'hydrogène, lorsque R' n'est pas un groupement amide.

A titre illustratif on peut citer parmi les divers radicaux:

en ce qui concerne R:

- les groupements méthyle;
- les groupements éthyle;
- les groupements propyle;
- les groupements isopropyle;
- les groupements butyle;
- les groupements isobutyle;
- les groupements $\alpha$-pentyle;
- les groupements t-butyle;
- les groupements chlorométhyle;
- les groupements dichlorométhyle;
- les groupements $\alpha$-chloro-éthyle;
- les groupements $\alpha,\beta$-dichloroéthyle;
- les groupements fluorométhyle;
- les groupements difluorométhyle;
- les groupements $\alpha,\beta$-difluoroéthyle;
- les groupements trifluoro-3,3,3 propyle;
- les groupements trifluoro cyclopropyle;
- les groupements trifluoro-4,4,4 butyle;
- les groupements heptafluoro-3,3,3,4,4,5,5 pentyle;
- les groupements $\beta$-cyanoéthyle;
- les groupements $\gamma$-cyanopropyle;
- les groupements phényle;
- les groupements p-chlorophényle;
- les groupements m-chlorophényle;
- les groupements dichloro-3,5 phényle;
- les groupements trichlorophényle;
- les groupements tétrachlorophényle;
- les groupements o-, p-, ou m-tolyle;
- les groupements $\alpha,\alpha,\alpha$-trifluorotolyle;
- les groupements xylyles (diméthyl-2,3 phényle; diméthyl-3,4 phényle).

Deux radicaux R peuvent par ailleurs constituer un radical méthylène, éthylène ou propylène.

en ce qui concerne R':

- un radical R ou $SiR_3$ tels que R représente l'un des divers groupements spécifiques définis page 6 lignes 3 à 32 ou un radical $-CO-(NR)-R'''$, R''' représentant R ou $SiR_3$, R représentant l'un des groupements respectivement définis dans ce paragraphe; R' pouvant constituer avec R'' un radical butylène, pentylène ou hexylène.

en ce qui concerne R'':

- l'hydrogène,
- les groupements méthyle,
- les groupements butyle,
- les groupements cyclohexyle,
- les groupements phényle,
- les groupements tolyle.

Préférentiellement la présente invention concerne un procédé d'obtention de composés à groupements isocyanuriques par catalyse aux composés aminosilylés de formule (I) dans laquelle les divers symboles représentent respectivement:

- R: un radical méthyle, éthyle, propyle, vinyle, phényle, ces radicaux peuvent éventuellement être chlorés et/ou fluorés,
- R': 
  - un radical alkyle choisi parmi les radicaux méthyle, éthyle, propyle ou butyle,
  - un radical $SiR_3$, R ayant l'une des significations définies lignes 18 à 20 page 7,
  - un radical carbonamide choisi parmi:
    $CO-NR-R$
    $-CO-NR-SiR_3$
    R ayant l'une des significations qui vient d'être données 18 à 20 page 7.

– R'': un radical méthyle, éthyle, propyle ou butyle, ou un atome d'hydrogène.

Enfin R' et R'' peuvent ensemble constituer un radical butylène ou pentylène.

Comme on l'a déjà dit le catalyseur peut être un aminosilane, un diaminosilane, une monosilylurée, une disilylurée ou un silazane. Il est facile déterminer les divers composés à groupements aminosilylés utilisables étant donné les diverses significations données précédemment aux divers radicaux R, R', R'', R'''. On notera en particulier que l'utilisation de silylurée obtenue par réaction d'amine secondaire et d'isocyanates-N silylé n'est pas envisagée. Ces silylurées sont impropres dans le procédé de l'invention puisque libérant l'isocyanate silylé lors du chauffage.

Les procédés d'obtention de composés à groupements isocyanuriques monomères ou polymères par cyclotrimérisation catalytique d'isocyanate dans lesquels le groupement isocyanate n'est pas directement relié à un atome de carbone appartenant à un groupement aromatique et qui utilisent en tant que catalyseur:

- un monoaminosilane (n étant égal à 1 et R' représentant un radical R, les radicaux R et R'' ayant l'une des significations préalablement données, deux radicaux R pouvant constituer ensemble un radical divalent ou bien encore R' et R'' pouvant constituer ensemble un radical divalent;
- un diaminosilane (n étant égal à 2, R' représentant un radical R, les radicaux R et R'' ayant l'une des significations préalablement données) ou 2 radicaux R pouvant constituer ensemble un radical divalent, ou encore 2 radicaux R' et R'' pouvant constituer ensemble un radical divalent;
- une silylurée (n étant égal à 1 et R' représentant un radical carbonamide

$$-\underset{\underset{O}{\|}}{C}-NRR'''$$

ou R''' représente un radical R ou $SiR_3$, les radicaux R et R'' ayant l'une des significations préalablement données, 2 radicaux R pouvant constituer ensemble un radical divalent ou les deux radicaux R' et R'' (R' représentant alors R) pouvant constituer ensemble un radical divalent;
- un silazane (n étant égal à 1 et R' représentant un groupement $SiR_3$),

constituent évidemment des objets plus spécifiques de la présente invention.

Parmi les aminosilanes ou diaminosilanes on citera:

- le méthylamino triméthylsilane;
- le diméthylamino triméthylsilane;
- le diéthylamino triméthylsilane;
- le dibutylamino triméthylsilane;
- le diéthylamino diméthylvinylsilane;
- le diéthylamino, diméthylphénylsilane;
- le bis diméthylamino, diméthylsilane;
- le bis diéthylamino, diméthylsilane;
- le bis dibutylamino diméthylsilane;
- le bis diméthylamino, méthylphénylsilane.

Parmi les silylurées on citera:

- la N méthyl N triméthylsilyl, N' méthyl N' butyl urée;
- la N triméthylsilyl N méthyl N', N' diméthyl urée;
- la N triméthylsilyl N éthyl N', N diméthyl urée;
- la N triméthylsilyl N butyl, N' butyl N' triméthylsilyl urée.

Les silazanes peuvent être symétriques ou dissymétriques; on emploie préférentiellement dans le cadre de la présente invention les disilazanes symétriques, les deux groupements $SiR_3$ étant identiques.

Parmi les disilazanes utilisables on citera:

- l'hexaméthyldisilazane,
- l'heptaméthyldisilazane,
- le diéthyl 1-3, tétraméthyl-1,1,3,3 disilazane,
- le divinyl-1,3 tétraméthyl-1,1,3,3 disilazane,
- l'hexaéthyldisilazane,
- le diphényl-1,3 tétraméthyl-1,1,3,3 disilazane.

Enfin on mentionnera tout particulièrement parmi les disilazanes l'hexaméthyldisilazane, l'heptaméthyldisilazane qui se révèlent être des catalyseurs tout particulièrement avantageux.

Le procédé selon l'invention permet de cyclotrimériser des mono ou polyisocyanates »simples« de nature aliphatique ou cycloaliphatique, ainsi que des polyisocyanates adducts (résultant de la polycondensation d'un excès de polyisocyanate sur un composé polyfonctionnel). La réaction de cyclotrimérisation peut être partielle ou totale: elle permet d'obtenir à partir de monoisocyanates des composés renfermant un seul groupement isocyanurique tel que les isocyanurates d'alkyle ou de cycloalkyle, et à partir de polyisocyanates simples ou adducts des polyisocyanates polyisocyanurates ainsi que des polyisocyanurates de masse moléculaire très élevées et ne renfermant plus de groupement isocyanate libre.

Tout isocyanate ou polyisocyanate simple de nature aliphatique ou cycloaliphatique ou tout isocyanate adduct ou prépolymère convient à condition comme on l'a déjà dit que le groupement isocyanate ne soit pas directement lié à un groupement aromatique.

Parmi les isocyanates utilisables on citera les monoisocyanates suivants:

- le méthylisocyanate,
- le butylisocyanate,
- le n-hexylisocyanate,
- le cyclohexylisocyanate,

Parmi les diisocyanates utilisables on citera:

- le tétraméthylène diisocyanate,
- le pentaméthylène diisocyanate,
- l'hexaméthylène diisocyanate,
- le diisocyanato-1,2 cyclohexane,
- le diisocyanato-1,4 cyclohexane,
- le bis(isocyanato-méthyl)-1,2 cyclobutane,
- le bis(isocyanato-4 cyclohexyl)-méthane,
- le triméthyl-3,3,5 isocyanatométhyl-5 isocyanato-1 cyclohexane.

Parmi ceux-ci on mentionnera tout particulièrement l'hexaméthylène diisocyanate.

Enfin on peut citer parmi les polyisocyanates adducts ou prépolymères utilisables les polyisocyanates modifiés qui sont ontenus en faisant réagir un excès de polyisocyanate aliphatique ou cycloaliphatique sur un composé comportant au moins deux groupements réactifs vis à vis des groupements isocyanates: tel qu'un diamine, un diacide ... Les polyisocyanates modifiés qui peuvent être mélangés avec des polyisocyanates simples peuvent comporter des groupements urée, biuret, ester, siloxane.

Bien que l'ordre d'introduction des réactifs ne soit pas critique le procédé selon l'invention est effectué avantageusement en introduisant dans l'isocyanate une faible quantité du catalyseur, celle-ci exprimée pondéralement par rapport à l'isocyanate engagé étant habituellement comprise entre 0,1 et 10% et de préférence entre 0,5 et 5% ; on peut éventuellement introduire de petites quantités supplémentaires de catalyseur durant la réaction.

Le procédé est effectué par simple chauffage des réactifs à une température en général comprise entre 50 et 180° et de préférence entre 80 et 130°C.

Lorsque la teneur en isocyanurate atteint la valeur désirée on peut détruire le catalyseur par exemple par addition d'un composé acide (acide fort tel que l'acide chlorhydrique, chlorure d'acide). Un tel procédé qui permet d'obtenir des polyisocyanates polyisocyanurates constitue également un objet de la présente invention.

On peut alors éventuellement éliminer le polyisocyanate monomère par tout moyen connu et parvenir à un polyisocyanate polyisocyanurate renfermant une teneur excessivement réduite en isocyanate monomère, ainsi qu'une faible teneur en dimère. De tels composés comme ceux issus d'hexaméthylène diisocyanate sont des composés bien connus et particulièrement intéressants comme constituants de base pour vernis et peinture.

Il est également possible, le cas échéant d'effectuer la réaction de cyclotrimérisation en milieu solvant, ce dernier pouvant être un solvant peu polaire comme par exemple un hydrocarbure aliphatique ou aromatique, ou un ester ou un éther on peut alors introduire le catalyseur dans le solvant et introduire dans cette solution l'isocyanate. On peut également introduire évidemment la solution catalytique dans l'isocyanate. Avantageusement le procédé est effectué sans solvant.

Les exemples qui suivent illustrent l'invention.

Exemple 1

Dans un ballon on charge:

- isocyanate de butyle: 39,6 g (soit 0,4 mole)

puis ajoute:

- N,N diméthyltriméthylsilylamine: 4,7 g (soit 0,04 mole)

On chauffe au bain d'huile jusqu'à 100–105°C dans la masse; après 14 heures à 100–110°C on a consommé 90% des NCO présents.

Par distillation on obtient 20,8 g d'un produit bouillant à 142°C sous 1 mm de mercure et identifié comme étant le tributylisocyanurate:

C % = 60,4 (théorie 60,6)
H % = 9,14 (théorie 9,1)
N % = 14,5 (théorie 14,15)
$n_D^{20}$ = 1,474

Absorption en infra rouge: à $1460^{cm^{-1}}$ et $1690^{cm^{-1}}$

Exemple 2

On procède comme pour l'exemple 1, à ceci près que l'on remplace le NN diméthyl triméthylsilylamine par 5,8 g de NN diéthyltriméthylsilylamine (0,04 mole).

Après 17 heures à 100–110°C on a consommé 88% des NCO présents. Par distillation on sépare 20,8 g de tributylisocyanurate, défini comme ci-dessus.

C % = 60,82 (théorie 60,6)
H % = 9,17 (théorie 9,1)
N % = 14,03 (théorie 14,15)

Exemple 3

Dans un ballon on charge:

– hexaméthylène diisocyanate 537,6 g (soit 3,2 moles)

On porte à 100°C et on coule dans l'isocyanate:

– NN diméthyltriméthylsilylamine 7,5 g (0,063 mole).

Après 1 heure 30 mn à 100°C on a consommé 15% des NCO présents.

On arrête la réaction par addition de 7 ml de chlorure de benzoyle. On distille la majorité de l'hexaméthylène diisocyanate restant jusqu'à 130° sous 1,5 mm.

Il reste alors 184 g de produit que l'on soumet à une distillation dans un appareil à film agité. On obtient 103 g de cyclotrimère titrant 0,546 NCO/100 g (théorie pour le trimère de l'hexaméthylène diisocyanate: 0,595) et ayant une viscosité de 16 poises à 25°C.

Exemple 4

Dans un ballon on charge:

– isocyanate de butyle 29,7 g (0,3 mole)
– bis (diméthylamino) diméthylsilane 0,88 g (0,006 mole)

On chauffe au bain d'huile à 105°C dans la masse; au bout de 14 heures à 105°C, on a consommé 88% des NCO présents.

Par distillation sous une pression de 0,5 m/m de mercure, on obtient 17,9 g de produits passant à 132–134°C et identifié comme étant le tributylisocyanurate.

Exemple 5

Comme dans l'exemple 4, on chauffe à 105°C un mélange constitué par:

– isocyanate de butyle 29,7 g (0,3 mole)
– triméthylsilylbutylamine 0,87 g

Après 23 heures à 105°C, 67% des NCO ont disparu.

Par distillation sous une pression de 0,5 m/m de mercure, on isole 15,7 g de produit bouillant entre 132 et 135°C, identifié comme étant le tributylisocyanurate.

Exemple 6

On introduit dans un ballon 29,7 g (0,3 mole) de butylisocyanate, puis coule à température ambiante en 10 minutes 1,45 g d'hexaméthyldisilazane (0,03 m). On porte à 100° pendant 2 heures, puis à 110°. Après 16 heures de réaction il ne reste que 7% des groupements isocyanates engagés.

On distille la masse réactionnelle et sépare 2,35 g de produits volatils; le résidu 13,35 g est le tributylisocyanurate caractérisé par les bandes d'absorption IR à 1465 et 1690 $cm^{-1}$ (on ne détecte ni bande urée, biuret ou carbodiimide). La microanalyse est conforme à la théorie.

| | dosé | | théorie |
|---|---|---|---|
| C % | 60,76 | 61,04 | 60,6 |
| H % | 9,1 | 9,48 | 9,1 |
| N % | 14,06 | 14,16 | 14,15 |

Exemple 7

On effectue un essai analogue à celui décrit à l'exemple 6 mais avec 2 moles d'hexaméthyldisilazane pour 100 moles de butylisocyanate. Après réaction il ne reste que 10% des groupements isocyanates engagés.

Le produit distillé fournit 20,75 g de tributylisocyanurate ($Eb_{0,2}$ 125°; PF 9—10°; $n_D^{20}$ = 1,4750).

On constate ainsi qu'une mole d'hexaméthyldisilazane amène la disparition de 90 moles de butylisocyanate.

Exemple 8

On introduit dans un ballon 134,4 g d'hexaméthylène diisocyanate (0,8 mole) et 1,3 g d'hexaméthyldisilazane (0,008 mole), puis chauffe à 100° durant 7 heures. La teneur en NCO est alors de 1,07 groupement par 100 g.

On distille l'excès d'hexaméthylène diisocyanate puis passe le résidu dans un évaporateur à film agité. On obtient ainsi 19 g de produit titrant 0,55 NCO/100 g (théorie pour le tris(isocyanato hexaméthylène) isocyanurate: 0,59).

Ce produit présente en IR les bandes caractéristique du trimère isocyanurique (1645 et 1690 $cm^{-1}$) et la bande NCO à 2270 $cm^{-1}$. Sa viscosité est de 25 poises à 25°C et son spectre en chromatographie GPC montre qu'il est essentiellement composé de trimère de PM 504. La teneur en dimère est inférieure à 5%. La teneur en hexaméthylène diisocyanate libre est inférieure à 0,1%.

Exemple 9

Dans un ballon tricol de 100 ml avec agitation magnétique, réfrigérant à reflux et arrivée d'azote, le chauffage étant assuré par un bain d'huile, on introduit:

- butylisocyanate: 24,75 g (0,25 mole)
- heptaméthyldisilazane: 4,4 g (0,025 mole)

On chauffe à 100—110°. Au bout de 3 heures, on a consommé la moitié des NCO présents. Après 16 heures de réaction, il ne reste que 8% des NCO, soit une consommation de 9,2 moles de butylisocyanate par mole d'heptaméthyldisilazane.

La distillation de la masse réactionnelle restante (22,2 g) fournit 14,45 g de produit passant à 112—132° sous 0,1 à 0,25 mm. Ce produit est du tributylisocyanurate ($n_D^{20}$ = 1,4760 — Spectre IR conforme).

**Revendications pour les Etats contractants: BE, CH, DE, FR, GB, IT, LI, LU, NL, SE**

1. Procédé d'obtention de composés à groupements isocyanuriques monomères ou polymères par cyclotrimérisation catalytique d'isocyanate dans lesquels le groupement isocyanate n'est pas directement relié à un atome de carbone appartenant à un groupement aromatique, la cyclotrimérisation étant effectuée par mise en contact de l'isocyanate et d'un composé initiant la réaction catalytique, le procédé étant caractérisé en ce que l'on utilise à titre de composé initiant la réaction catalytique un composé à groupement aminosilylé et ayant pour formule (I):

$$R_{(4-n)}-Si-[NR'R'']_n \qquad (I)$$

dans laquelle les divers symboles représentent respectivement:

- R: un radical monovalent de nature hydrocarbonée, aliphatique, cycloaliphatique saturé ou insaturé, aryle, aralkyle ou alkylaryle, éventuellement substitué par des atomes d'halogène ou des groupements CN deux radicaux R pouvant constituer ensemble un radical hydrocarboné divalent,
- R': un radical monovalent choisi parmi les radicaux R, $SiR_3$ ou les radicaux amide de formule:

$$\begin{array}{l} -CO-N-R''' \\ \qquad\quad | \\ \qquad\quad R \end{array}$$

  R''' représentant R ou $SiR_3$, R ayant la signification préalablement donnée, le radical R' pouvant éventuellement lorsqu'il ne représente pas un groupement amide ou un groupement $SiR_3$ constituer avec le radical R'' un radical hydrocarboné divalent,
- R'': un radical monovalent ayant la même signification que le radical R, ou un atome d'hydrogène lorsque R' n'est pas un radical amide,
- n: un nombre entier égal à 1 ou 2. Lorsque n est égal à 2, R' est un radical R.

2. Procédé selon la revendication 1 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un composé à groupement aminosilylé de formule (I)

$$R_{(4-n)}-Si-[NR'R'']_n$$

dans laquelle les divers symboles représentent respectivement

- R: un radical alkyle, alkényle ou halogénoalkyle ou halogénoalkényle ayant de 1 à 5 atomes de carbone et comportant de 1 à 6 atomes de chlore et/ou de fluor des radicaux cycloalkyle, cycloalkényle et halogénocycloalkyle, halogénocycloalkényle ayant de 3 à 8 atomes de carbone et contenant de 1 à 4 atomes de chlore et/ou de fluor
  des radicaux aryles, alkylaryles et halogénoaryles ayant de 6 à 8 atomes de carbone et contenant de 1 à 4 atomes de chlore et/ou de fluor
  des radicaux cyanoalkyles ayant de 3 à 4 atomes de carbone
- deux symboles R portés par un même atome de silicium constituent entre eux un radical divalent ayant de 1 à 4 atomes de carbone.
- R': un radical monovalent choisi par les radicaux R, $SiR_3$, et CO(NR)—R''', R''' représentant R ou $SiR_3$, R ayant la signification plus précise qui vient juste d'être donnée dans la revendication 2; R' pouvant constituer avec R'' un radical alkylène ayant de 4 à 6 atomes de carbone.
- R'': un radical alkyle ou alkényle ayant de 1 à 4 atomes de carbone, un radical cycloalkyle ou cycloalkényle ayant de 4 à 6 atomes de carbone nucléaires, un radical phényle ou tolyle ou xylyle, ou un atome d'hydrogène, lorsque R' n'est pas un groupement amide.

3. Procédé selon l'une des revendications 1 ou 2 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un composé à groupement aminosilylé de formule (I)

$$R_{(4-n)}-Si-[NR'R'']_n$$

dans laquelle les divers symboles représentent respectivement

– R: un radical méthyle, éthyle, propyle, vinyle, phényle, ces radicaux peuvent éventuellement être chlorés et/ou fluorés,
– R': – un radical alkyle choisi parmi les radicaux méthyle, éthyle, propyle ou butyle,
  – un radical $SiR_3$, R ayant la signification donnée dans la revendication 3,
  – un radical carbonamide choisi parmi:
  $CO-NR-R$
  $-CO-NR-SiR_3$
  R ayant l'une des significations spécifiques qui vient d'être donnée dans cette revendication.
– R": un radical méthyle, éthyle, propyle ou butyle, ou un atome d'hydrogène.

Enfin R' et R" peuvent ensemble constituer un radical butylène ou pentylène.

4. Procédé selon l'une des revendications précédentes pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un aminosilane $R_3Si\ NR'R''$ les divers symboles ayant l'une des significations préalablement données.

5. Procédé selon l'une des revendications 1 à 3 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un diaminosilane $R_2Si(NR'R'')_2$, les divers symboles ayant l'une des significations préalablement données.

6. Procédé selon l'une des revendications 1 à 3 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un silylurée

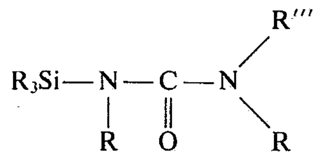

les divers symboles ayant l'une des significations préalablement données.

7. Procédé selon l'une des revendications 1 à 3 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un silazane

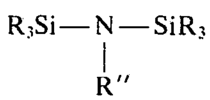

les divers symboles ayant l'une des significations préalablement données.

8. Procédé selon l'une des revendications 1 à 7 dans lequel on obtient un polyisocyanate polyisocyanurate par cyclotrimérisation catalytique de polyisocyanate aliphatique, cycloaliphatique, adduct ou prépolymère et selon lequel détruit le catalyseur lorsque la teneur désirée en isocyanurate est atteinte.

9. Procédé selon la revendication 8 dans lequel la destruction du catalyseur est réalisée par addition d'un composé acide.

10. Procédé d'obtention de polyisocyanate polyisocyanurate par cyclotrimérisation de diisocyanate aliphatique ou cycloaliphatique selon l'une des revendications 8 et 9, caractérisé en ce que l'on élimine en fin de réaction le diisocyanate monomère résiduel.

11. Procédé selon l'une des revendications 1 à 10 dans lequel le polyisocyanate est l'hexaméthylène diisocyanate.

12. Composés à groupements isocyanuriques obtenus selon l'une des revendications 1 à 11.

**Revendications pour l'Etat contractant: AT**

1. Procédé d'obtention de composés à groupements isocyanuriques monomères ou polymères par cyclotrimérisation catalytique d'isocyanate dans lesquels le groupement isocyanate n'est pas directement relié à un atome de carbone appartenant à un groupement aromatique, la cyclotrimérisation étant

effectuée par mise en contact de l'isocyanate et d'un composé initiant la réaction catalytique, le procédé étant caractérisé en ce que l'on utilise à titre de composé initiant la réaction catalytique un composé à groupement aminosilylé et ayant pour formule (I):

$$R_{(4-n)}Si-[NR'R'']_n \qquad (I)$$

dans laquelle les divers symboles représentent respectivement:

- R: un radical monovalent de nature hydrocarbonée, aliphatique, cycloaliphatique saturé ou insaturé, aryle, aralkyle ou alkylaryle, éventuellement substitué par des atomes d'halogène ou des groupements CN deux radicaux R pouvant constituer ensemble un radical hydrocarboné divalent,
- R': un radical monovalent choisi parmi les radicaux R, $SiR_3$ ou les radicaux amide de formule:

$$-CO-\underset{\displaystyle R}{\underset{|}{N}}-R'''$$

  R''' représentant R ou $SiR_3$, R ayant la signification préalablement donnée, le radical R' pouvant éventuellement lorsqu'il ne représente pas un groupement amide ou un groupement $SiR_3$ constituer avec le radical R'' un radical hydrocarboné divalent,
- R'': un radical monovalent ayant la même signification que le radical R, ou un atome d'hydrogène lorsque R' n'est pas un radical amide,
- n: un nombre entier égal à 1 ou 2. Lorsque n est égal à 2, R' est un radical R.

2. Procédé selon la revendication 1 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un composé à groupement aminosilylé de formule (I)

$$R_{(4-n)}Si-[NR'R'']_n$$

dans laquelle les divers symboles représentent respectivement

- R: un radical alkyle, alkényle ou halogénoalkyle ou halogénoalkényle ayant de 1 à 5 atomes de carbone et comportant de 1 à 6 atomes de chlore et/ou de fluor des radicaux cycloalkyle, cycloalkényle et halogénocycloalkyle, halogénocycloalkényle ayant de 3 à 8 atomes de carbone et contenant de 1 à 4 atomes de chlore et/ou de fluor
  des radicaux aryles, alkylaryles et halogénoaryles ayant de 6 à 8 atomes de carbone et contenant de 1 à 4 atomes de chlore et/ou de fluor
  des radicaux cyanoalkyles ayant de 3 à 4 atomes de carbone
- deux symboles R portés par un même atome de silicium constituent entre eux un radical divalent ayant de 1 à 4 atomes de carbone.
- R': un radical monovalent choisi par les radicaux R, $SiR_3$, et CO(NR)—R''', R''' représentant R ou $SiR_3$, R ayant la signification plus précise qui vient juste d'être donnée dans la revendication 2; R' pouvant constituer avec R'' un radical alkylène ayant de 4 à 6 atomes de carbone.
- R'': un radical alkyle ou alkényle ayant de 1 à 4 atomes de carbone, un radical cycloalkyle ou cycloalkényle ayant de 4 à 6 atomes de carbone nucléaires, un radical phényle ou tolyle ou xylyle, ou un atome d'hydrogène, lorsque R' n'est pas un groupement amide.

3. Procédé selon l'une des revendications 1 ou 2 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un composé à groupement aminosilylé de formule (I)

$$R_{(4-n)}Si-[NR'R'']_n$$

dans laquelle les divers symboles représentent respectivement

- R: un radical méthyle, éthyle, propyle, vinyle, phényle, ces radicaux peuvent éventuellement être chlorés et/ou fluorés,
- R': – un radical alkyle choisi parmi les radicaux méthyle, éthyle, propyle ou butyle,
  – un radical $SiR_3$, R ayant la signification donnée dans la revendication 3,

- un radical carbonamide choisi parmi:
  CO−NR−R
  −CO−NR−$SiR_3$
  R ayant l'une des significations spécifiques qui vient d'être donnée dans cette revendication.
- R'': un radical méthyle, éthyle, propyle ou butyle, ou un atome d'hydrogène.

Enfin R' et R'' peuvent ensemble constituer un radical butylène ou pentylène.

4. Procédé selon l'une des revendications précédentes pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un aminosilane $R_3Si$ NR'R'' les divers symboles ayant l'une des significations préalablement données.

5. Procédé selon l'une des revendications 1 à 3 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un diaminosilane $R_2Si(NR'R'')_2$, les divers symboles ayant l'une des significations préalablement données.

6. Procédé selon l'une des revendications 1 à 3 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un silylurée

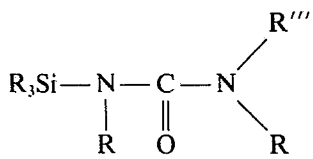

les divers symboles ayant l'une des significations préalablement données.

7. Procédé selon l'une des revendications 1 à 3 pour l'obtention de composés à groupements isocyanuriques monomères ou polymères par cyclotrimérisation catalytique d'isocyanates dans lesquels le groupement isocyanate n'est pas directement relié à un atome de carbone appartenant à un groupement aromatique caractérisé en ce que l'on utilise à titre de composé initiant la réaction catalytique un silazane

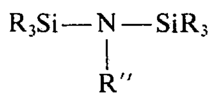

les divers symboles ayant l'une des significations préalablement données.

8. Procédé selon l'une des revendications 1 à 7 dans lequel on obtient un polyisocyanate polyisocyanurate par cyclotrimérisation catalytique de polyisocyanate aliphatique, cycloaliphatique, adduct ou prépolymère et selon lequel détruit le catalyseur lorsque la teneur désirée en isocyanurate est atteinte.

9. Procédé selon la revendication 8 dans lequel la destruction du catalyseur est réalisée par addition d'un composé acide.

10. Procédé d'obtention de polyisocyanate polyisocyanurate par cyclotrimérisation de diisocyanate aliphatique ou cycloaliphatique selon l'une des revendications 8 et 9, caractérisé en ce que l'on élimine en fin de réaction le diisocyanate monomère résiduel.

11. Procédé selon l'une des revendications 1 à 10 dans lequel le polyisocyanate est l'hexaméthylène diisocyanate.

**Patentansprüche für die Vertragsstaaten: BE, CH, DE, FR, GB, IT, LI, LU, NL, SE**

1. Verfahren zur Herstellung von Verbindungen mit monomeren oder polymeren Isocyanidgruppen durch katalytische Cyclotrimerisation von Isocyanat, worin die Isocyanatgruppe nicht direkt an ein aus einer aromatischen Gruppe stammendes Kohlenstoffatom gebunden ist, wobei die Cyclotrimerisation ausgeführt wird, indem man das Isocyanat und eine die katalytische Reaktion einleitende Verbindung in Kontakt bringt, welches Verfahren dadurch gekennzeichnet ist, daß ma als die katalytische Reaktion einleitende Verbindung eine Verbindung mit Aminosilylgruppe der Formel (I):

$$R_{(4-n)} - Si - [NR'R'']_n \qquad (I)$$

einsetzt, in welcher die verschiedenen Symbole jeweils darstellen:

- R: einen einwertigen Rest vom Typ gesättigter oder ungesättigter, aliphatischer oder cycloaliphatischer Kohlenwasserstoff, Aryl, Aralkyl oder Alkylaryl, gegebenenfalls substituiert durch Halogenatome oder CN-Gruppen, wobei zwei Reste R zusammen einen zweiwertigen Kohlenwasserstoffrest bilden können,
- R': einen einwertigen Rest, ausgewählt aus den Resten R, $SiR_3$ oder den Amidresten der Formel:

$$\begin{array}{c} -CO-N-R''' \\ \quad\;\; | \\ \quad\;\; R \end{array}$$

  wobei R''' R oder $SiR_3$ darstellt, R die zuvor angegebene Bedeutung hat, der Rest R' gegebenenfalls, wenn er nicht eine Amidgruppe oder eine Gruppe $SiR_3$ darstellt, mit dem Rest R'' eine zweiwertige Kohlenwasserstoffgruppe bilden kann,
- R'': einen einwertigen Rest, der die gleiche Bedeutung wie der Rest R hat, oder ein Wasserstoffatom, wenn R' kein Amidrest ist,
- n: eine ganze Zahl gleich 1 oder 2. Wenn n gleich 2 ist, ist R' ein Rest R.

2. Verfahren nach Anspruch 1 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung eine Verbindung mit Aminosilylgruppe der Formel (I)

$$R_{(4-n)}-Si-[NR'R'']_n$$

einsetzt, in welcher die verschiedenen Symbole jeweils darstellen:

- R: einen Alkyl-, Alkenyl- oder Halogenalkyl- oder Halogenalkenylrest mit 1 bis 5 Kohlenstoffatomen, der 1 bis 6 Chlor- und/oder Fluoratome enthält, Cycloalkyl-, Cycloalkenyl- und Halogencycloalkyl-, Halogencycloalkenylreste mit 3 bis 8 Kohlenstoffatomen, die 1 bis 4 Chlor- und/oder Fluoratome enthalten,
  Aryl-, Alkylaryl- und Halogenarylreste mit 6 bis 8 Kohlenstoffatomen, die 1 bis 4 Chlor- und/oder Fluoratome enthalten,
  Cyanoalkylreste mit 3 bis 4 Kohlenstoffatomen,
- zwei Symbole R, die an das gleiche Siliciumatom gebunden sind, miteinander einen zweiwertigen Rest mit 1 bis 4 Kohlenstoffatomen bilden;
- R': einen einwertigen Rest, ausgewählt aus den Resten R, $SiR_3$, und CO(NR)−R''', wobei R''' R oder $SiR_3$ darstellt, R die speziellere Bedeutung hat, die zuvor im Anspruch 2 angegeben wurde; R' mit R'' einen Alkylenrest mit 4 bs 6 Kohlenstoffatomen bilden kann;
- R'': einen Alkyl- oder Alkenylrest mit 1 bis 4 Kohlenstoffatomen, einen Cycloalkyl- oder Cycloalkenylrest mit 4 bis 6 Ringkohlenstoffatomen, einen Phenyl- oder Tolyl- oder Xylylrest, oder ein Wasserstoffatom, wenn R' keine Amidgruppe ist.

3. Verfahren nach einem der Ansprüche 1 oder 2 zur Herstellung von Verbindungen mit monomeren oder polymeren Isocyanidgruppen durch katalytische Cyclotrimerisation von Isocyanaten, in welchen die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung eine Verbindung mit Aminosilylgruppe der Formel (I)

$$R_{(4-n)}-Si-[NR'R'']_n$$

einsetzt, in welcher die verschiedenen Symbole jeweils darstellen:

- R: einen Methyl-, Äthyl-, Propyl-, Vinyl-, Phenylrest, welche Reste gegebenenfalls chloriert und/oder fluoriert sein können,
- R':
  - einen Alkylrest, ausgewählt aus den Methyl-, Äthyl-, Propyl- oder Butylresten,
  - einen Rest $SiR_3$, wobei R die in Anspruch 3 angegebene Bedeutung hat,
  - einen Carbonamidrest, ausgewählt aus:
    CO−NR−R
    $-CO-NR-SiR_3$
    wobei R eine der speziellen Bedeutungen hat, die in diesem Anspruch angegeben wurden,
- R'': einen Methyl-, Äthyl-, Propyl- oder Butylrest oder ein Wasserstoffatom;

schließlich können R' und R'' zusammen einen Butylen- oder Pentylenrest bilden.

4. Verfahren nach einem der vorhergehenden Ansprüche zur Herstellung von Verbindungen mit monomeren oder polymeren Isocyanidgruppen durch katalytische Cyclotrimerisation von Isocyanaten, in welchen die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Aminosilan $R_3Si\ NR'R''$ einsetzt, dessen verschiedenen Symbole eine der zuvor angegebenen Bedeutungen haben.

5. Verfahren nach einem der Ansprüche 1 bis 3 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Diaminosilan $R_2Si(NR'R'')_2$ verwendet, dessen verschiedenen Symbole eine der zuvor angegebenen Bedeutungen haben.

6. Verfahren nach einem der Ansprüche 1 bis 3 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Silylcarbamid

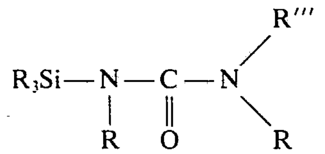

einsetzt, wobei die verschiedenen Symbole eine der zuvor angegebenen Bedeutungen haben.

7. Verfahren nach einem der Ansprüche 1 bis 3 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Silazan

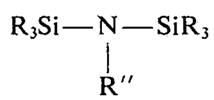

einsetzt, worin die verschiedenen Symbole eine der zuvor angegebenen Bedeutungen haben.

8. Verfahren nach einem der Ansprüche 1 bis 7, in welchem man ein Polyisocyanurat-polyisocyanat durch katalytische Cyclotrimerisation von aliphatischem oder cycloaliphatischem Polyisocyanataddukt oder -präpolymer erhält und bei welchem man den Katalysator zerstört, wenn der gewünschte Gehalt an Isocyanurat erreicht ist.

9. Verfahren nach Anspruch 8, bei welchem die Zerstörung des Katalysators durch Zugabe einer sauren Verbindung ausgeführt wird.

10. Verfahren zur Herstellung von Polyisocyanurat-polyisocyanat durch Cyclotrimerisation von aliphatischem oder cycloaliphatischem Diisocyanat nach einem der Ansprüche 8 und 9, dadurch gekennzeichnet, daß man am Ende der Reaktion das restliche monomere Diisocyanat entfernt.

11. Verfahren nach einem der Ansprüche 1 bis 10, in welchem das Polyisocyanat Hexamethylendiisocyanat ist.

12. Verbindungen mit Isocyanidgruppen, erhalten nach einem der Ansprüche 1 bis 11.

**Patentansprüche für den Vertragsstaat: AT**

1. Verfahren zur Herstellung von Verbindungen mit monomeren oder polymeren Isocyanidgruppen durch katalytische Cyclotrimerisation von Isocyanat, worin die Isocyanatgruppe nicht direkt an ein aus einer aromatischen Gruppe stammendes Kohlenstoffatom gebunden ist, wobei die Cyclotrimerisation ausgeführt wird, indem man das Isocyanat und eine die katalytische Reaktion einleitende Verbindung in Kontakt bringt, welches Verfahren dadurch gekennzeichnet ist, daß ma als die katalytische Reaktion einleitende Verbindung eine Verbindung mit Aminosilylgruppe der Formel (I):

$$R_{(4-n)}\ Si - [NR'R'']_n \qquad (I)$$

einsetzt, in welcher die verschiedenen Symbole jeweils darstellen:

– R: einen einwertigen Rest vom Typ gesättigter oder ungesättigter, aliphatischer oder cycloaliphatischer Kohlenwasserstoff, Aryl, Aralkyl oder Alkylaryl, gegebenenfalls substituiert durch Halogenatome oder CN-Gruppen, wobei zwei Reste R zusammen einen zweiwertigen Kohlenwasserstoffrest bilden können,

– R': einen einwertigen Rest, ausgewählt aus den Resten R, $SiR_3$ oder den Amidresten der Formel:

$$\begin{array}{l} -CO-N-R''' \\ \qquad\quad\; | \\ \qquad\quad\; R \end{array}$$

wobei R''' R oder $SiR_3$ darstellt, R die zuvor angegebene Bedeutung hat, der Rest R' gegebenenfalls, wenn er nicht eine Amidgruppe oder eine Gruppe $SiR_3$ darstellt, mit dem Rest R'' eine zweiwertige Kohlenwasserstoffgruppe bilden kann,

– R'': einen einwertigen Rest, der die gleiche Bedeutung wie der Rest R hat, oder ein Wasserstoffatom, wenn R' kein Amidrest ist,

– n: eine ganze Zahl gleich 1 oder 2. Wenn n gleich 2 ist, ist R' ein Rest R.

2. Verfahren nach Anspruch 1 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung eine Verbindung mit Aminosilylgruppe der Formel (I)

$$R_{(4-n)}-Si-[NR'R'']_n$$

einsetzt, in welcher die verschiedenen Symbole jeweils darstellen:

– R: einen Alkyl-, Alkenyl- oder Halogenalkyl- oder Halogenalkenylrest mit 1 bis 5 Kohlenstoffatomen, der 1 bis 6 Chlor- und/oder Fluoratome enthält, Cycloalkyl-, Cycloalkenyl- und Halogencycloalkyl-, Haolgencycloalkenylreste mit 3 bis 8 Kohlenstoffatomen, die 1 bis 4 Chlor- und/oder Fluoratome enthalten,
Aryl-, Alkylaryl- und Halogenarylreste mit 6 bis 8 Kohlenstoffatomen, die 1 bis 4 Chlor- und/oder Fluoratome enthalten,
Cyanoalkylreste mit 3 bis 4 Kohlenstoffatomen,

– zwei Symbole R, die an das gleiche Siliciumatom gebunden sind, miteinander einen zweiwertigen Rest mit 1 bis 4 Kohlenstoffatomen bilden;

– R': einen einwertigen Rest, ausgewählt aus den Resten R, $SiR_3$, und CO(NR)–R''', wobei R''' R oder $SiR_3$ darstellt, R die speziellere Bedeutung hat, die zuvor im Anspruch 2 angegeben wurde; R' mit R'' einen Alkylenrest mit 4 bis 6 Kohlenstoffatomen bilden kann;

– R'': einen Alkyl- oder Alkenylrest mit 1 bis 4 Kohlenstoffatomen, einen Cycloalkyl- oder Cycloalkenylrest mit 4 bis 6 Ringkohlenstoffatomen, einen Phenyl- oder Tolyl- oder Xylylrest, oder ein Wasserstoffatom, wenn R' keine Amidgruppe ist.

3. Verfahren nach einem der Ansprüche 1 oder 2 zur Herstellung von Verbindungen mit monomeren oder polymeren Isocyanidgruppen durch katalytische Cyclotrimerisation von Isocyanaten, in welchen die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung eine Verbindung mit Aminosilylgruppe der Formel (I)

$$R_{(4-n)}-Si-[NR'R'']_n$$

einsetzt, in welcher die verschiedenen Symbole jeweils darstellen:

– R: einen Methyl-, Äthyl-, Propyl-, Vinyl-, Phenylrest, welche Reste gegebenenfalls chloriert und/oder fluoriert sein können,

– R': – einen Alkylrest, ausgewählt aus den Methyl-, Äthyl-, Propyl- oder Butylresten,
  – einen Rest $SiR_3$, wobei R die in Anspruch 3 angegebene Bedeutung hat,
  – einen Carbonamidrest, ausgewählt aus:
  CO–NR–R
  –CO–NR–$SiR_3$
  wobei R eine der speziellen Bedeutungen hat, die in diesem Anspruch angegeben wurden

– R'': einen Methyl-, Äthyl-, Propyl- oder Butylrest oder ein Wasserstoffatom;

schließlich können R' und R'' zusammen einen Butylen- oder Pentylenrest bilden.

4. Verfahren nach einem der vorhergehenden Ansprüche zur Herstellung von Verbindungen mit monomeren oder polymeren Isocyanidgruppen durch katalytische Cyclotrimerisation von Isocyanaten,

in welchen die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Aminosilan $R_3Si\ NR'R''$ einsetzt, dessen verschiedenen Symbole eine der zuvor angegebenen Bedeutungen haben.

5. Verfahren nach einem der Ansprüche 1 bis 3 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Diaminosilan $R_2Si(NR'R'')_2$ verwendet, dessen verschiedenen Symbole eine der zuvor angegebenen Bedeutungen haben.

6. Verfahren nach einem der Ansprüche 1 bis 3 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Silylcarbamid

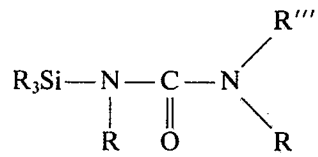

einsetzt, wobei die verschiedenen Symbole eine der zuvor angegebenen Bedeutungen haben.

7. Verfahren nach einem der Ansprüche 1 bis 3 zur Herstellung von Verbindungen mit monomeren oder polymeren Isocyanidgruppen durch katalytische Cyclotrimerisation von Isocyanaten, worin die Isocyanatgruppe nicht direkt an ein aus einer aromatischen Gruppe stammendes Kohlenstoffatom gebunden ist, dadurch gekennzeichnet, daß man als die katalytische Reaktion einleitende Verbindung ein Silazan

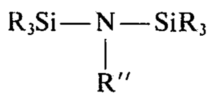

einsetzt, worin die verschiedenen Symbole eine der zuvor angegebenen Bedeutungen haben.

8. Verfahren nach einem der Ansprüche 1 bis 7, in welchem man ein Polyisocyanurat-polyisocyanat durch katalytische Cyclotrimerisation von aliphatischem oder cycloaliphatischem Polyisocyanataddukt oder -präpolymer erhält und bei welchem man den Katalysator zerstört, wenn der gewünschte Gehalt an Isocyanurat erreicht ist.

9. Verfahren nach Anspruch 8, bei welchem die Zerstörung des Katalysators durch Zugabe einer sauren Verbindung ausgeführt wird.

10. Verfahren zur Herstellung von Polyisocyanurat-polyisocyanat durch Cyclotrimerisation von aliphatischem oder cycloaliphatischem Diisocyanat nach einem der Ansprüche 8 und 9, dadurch gekennzeichnet, daß man am Ende der Reaktion das restliche monomere Diisocyanat entfernt.

11. Verfahren nach einem der Ansprüche 1 bis 10, in welchem das Polyisocyanat Hexamethylendiisocyanat ist.

**Claims for the contracting states: BE, CH, DE, FR, GB, IT, LI, LU, NL, SE**

1. Process for obtaining monomeric or polymeric compounds having isocyanurate groups by catalytic cyclotrimerisation of an isocyanate in which the isocyanate group is not directly bonded to a carbon atom belonging to an aromatic group, the cyclotrimerisation being carried out by bringing the isocyanate into contact with a compound which initiates the catalytic reaction and the process being characterised in that there is used, as the compound initiating the catalytic reaction, a compound possessing an aminosilyl group and having the formula (I):

$$R_{(4-n)}-Si-[NR'R'']_n \qquad (I)$$

in which the various symbols respectively represent the following:

R: a monovalent hydrocarbon radical which may be an aliphatic, saturated or unsaturated cycloaliphatic, aryl, aralkyl or alkylaryl radical, optionally substituted by halogen atoms or CN groups, two radicals R being able conjointly to form a divalent hydrocarbon radical,

R': a monovalent radical chosen from among the radicals R, $SiR_3$ or the amide radicals of the formula:

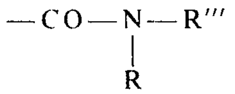

where R''' represents R or $SiR_3$, R having the meaning given above, and the radical R', if it does not represent an amide group or $SiR_3$ group, optionally being able to form, conjointly with the radical R'', a divalent hydrocarbon radical,

R'': a monovalent radical having the same meaning as the radical R, or a hydrogen atom if R' is not an amide radical,

n: an integer equal to 1 or 2, with R' being a radical R if n is equal to 2.

2. Process according to Claim 1 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a compound, possessing an aminosilyl group, of the formula (I)

$$R_{(4-n)}Si-[NR'R'']_n$$

in which the various symbols respectively represent the following:

R: an alkyl, alkenyl, halogenoalkyl or halogenoalkenyl radical having from 1 to 5 carbon atoms and containing from 1 to 6 chlorine and/or fluorine atoms, cycloalkyl, cycloalkenyl, halogenocycloalkyl and halogenocycloalkenyl radical having from 3 to 8 carbon atoms and containing from 1 to 4 chlorine and/or fluorine atoms, aryl, alkylaryl and halogenoaryl radicals having from 6 to 8 carbon atoms and containing from 1 to 4 chlorine and/or fluorine atoms and cyanoalkyl radical having from 3 to 4 carbon atoms, two symbols R carried on one and the same silicon atom together forming optionaly a divalent radical having from 1 to 4 carbon atoms,

R': a monovalent radical chosen from among the radicals R, $SiR_3$ and CO(NR)–R''', R''' representing R or $SiR_3$ and R having the more precise meaning which has just been given in Claim 2, and R' and R'' conjointly being able to form an alkylene radical having from 4 to 6 carbon atoms,

R'': an alkyl or alkenyl radical having from 1 to 4 carbon atoms, a cycloalkyl or cycloalkenyl radical having from 4 to 6 nuclear carbon atoms, a phenyl, tolyl or xylyl radical, or a hydrogen atom if R' is not an amide group.

3. Process according to one of Claims 1 or 2 for obtaining monomeric or polymeric compounds having isocyanurate groups by catalytic cyclotrimerisation of isocyanates in which the isocyanate group is not directly bonded to a carbon atom belonging to an aromatic group, characterised in that, as the compound initiating the catalytic reaction there is used a compound having an aminosilyl group, of the formula (I)

$$R_{(4-n)}Si-[NR'R'']_n$$

in which the various symbols respectively represent the following:

R: a methyl, ethyl, propyl, vinyl or phenyl radical, these radicals being optionally chlorinated and/or fluorinated,

R': an alkyl radical chosen from among methyl, ethyl, propyl or butyl radicals, an $SiR_3$ radical, R having the meaning given in Claim 3, or a carbonamide radical chosen from among CO–NR–R and –CO–NR–$SiR_3$, R having one of the specific meanings which have just been given in this claim,

R'': a methyl, ethyl, propyl or butyl radical or a hydrogen atom,

and in which, finally, R' and R'' can together form a butylene or pentylene radical.

4. Process according to one of the preceding claims for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used an aminosilane $R_3Si\ NR'R''$, the various symbols having one of the meanings given above.

5. Process according to one of Claims 1 to 3 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a diaminosilane $R_2Si(NR'R'')_2$, the various symbols having one of the meanings given above.

6. Process according to one of Claims 1 to 3 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a silylurea

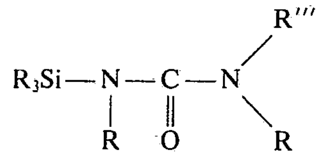

the various symbols having one of the meanings given above.

7. Process according to one of Claims 1 to 3 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a silazane

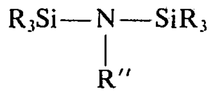

the various symbols having one of the meanings given above.

8. Process according to one of Claims 1 to 7 in which a polyisocyanate-polyisocyanurate is obtained by catalytic cyclotrimerisation of an aliphatic or cycloaliphatic polyisocyanate or an adduct or prepolymer and in which the catalyst is destroyed when the desired isocyanurate content has been reached.

9. Process according to Claim 8 in which the catalyst is destroyed by adding an acid compound.

10. Process for obtaining a polyisocyanate-polyisocyanurate by cyclotrimerisation of an aliphatic or cycloaliphatic diisocyanate according to one of Claims 8 and 9, characterised in that at the end of the reaction the residual monomeric diisocyanate is removed.

11. Process according to one of Claims 1 to 10 in which the polyisocyanate is hexamethylene diisocyanate.

12. Compounds having isocyanurate groups, obtained according to one of Claims 1 to 11.

**Claims for the contracting state: AT**

1. Process for obtaining monomeric or polymeric compounds having isocyanurate groups by catalytic cyclotrimerisation of an isocyanate in which the isocyanate group is not directly bonded to a carbon atom belonging to an aromatic group, the cyclotrimerisation being carried out by bringing the isocyanate into contact with a compound which initiates the catalytic reaction and the process being characterised in that there is used, as the compound initiating the catalytic reaction, a compound possessing an aminosilyl group and having the formula (I):

$$R_{(4-n)}\text{—}Si\text{—}[NR'R'']_n \qquad (I)$$

in which the various symbols respectively represent the following:

R: a monovalent hydrocarbon radical which may be an aliphatic, saturates or unsaturated cycloaliphatic, aryl, aralkyl or alkylaryl radical, optionally substituted by halogen atoms or CN groups, two radicals R being able conjointly to form a divalent hydrocarbon radical,

R': a monovalent radical chosen from among the radicals R, $SiR_3$ or the amide radicals of the formula:

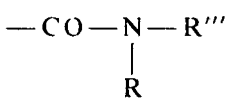

where R''' represents R or $SiR_3$, R having the meaning given above, and the radical R', if it does not represent an amide group or $SiR_3$ group, optionally being able to form, conjointly with the radical R'', a divalent hydrocarbon radical,

R'': a monovalent radical having the same meaning as the radical R, or a hydrogen atom if R' is not an amide radical,

n: an integer equal to 1 or 2, with R' being a radical R if n is equal to 2.

2. Process according to Claim 1 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a compound, possessing an aminosilyl group, of the formula (I)

$$R_{(4-n)}Si-[NR'R'']_n$$

in which the various symbols respectively represent the following:

R: an alkyl, alkenyl, halogenoalkyl or halogenoalkenyl radical having from 1 to 5 carbon atoms and containing from 1 to 6 chlorine and/or fluorine atoms, cycloalkyl, cycloalkenyl, halogenocycloalkyl and halogenocycloalkenyl radical having from 3 to 8 carbon atoms and containing from 1 to 4 chlorine and/or fluorine atoms, aryl, alkylaryl and halogenoaryl radical having from 6 to 8 carbon atoms and containing from 1 to 4 chlorine and/or fluorine atoms and cyanoalkyl radical having from 3 to 4 carbon atoms, two symbols R carried on one and the same silicon atom together forming optionaly a divalent radical having from 1 to 4 carbon atoms,
R': a monovalent radical chosen from among the radicals R, $SiR_3$ and CO(NR)−R''', R''' representing R or $SiR_3$ and R having the more precise meaning which has just been given in Claim 2, and R' and R'' conjointly being able to form an alkylene radical having from 4 to 6 carbon atoms,
R'': an alkyl or alkenyl radical having from 1 to 4 carbon atoms, a cycloalkyl or cycloalkenyl radical having from 4 to 6 nuclear carbon atoms, a phenyl, tolyl or xylyl radical, or a hydrogen atom if R' is not an amide group.

3. Process according to one of Claims 1 or 2 for obtaining monomeric or polymeric compounds having isocyanurate groups by catalytic cyclotrimerisation of isocyanates in which the isocyanate group is not directly bonded to a carbon atom belonging to an aromatic group, characterised in that, as the compound initiating the catalytic reaction there is used a compound having an aminosilyl group, of the formula (I)

$$R_{(4-n)}Si-[NR'R'']_n$$

in which the various symbols respectively represent the following:

R: a methyl, ethyl, propyl, vinyl or phenyl radical, these radicals being optionally chlorinated and/or fluorinated,
R': an alkyl radical chosen from among methyl, ethyl, propyl or butyl radicals, an $SiR_3$ radical, R having the meaning given in Claim 3, or a carbonamide radical chosen from among CO−NR−R and −CO−NR−$SiR_3$, R having one of the specific meanings which have just been given in this claim,
R'': a methyl, ethyl, propyl or butyl radical or a hydrogen atom,

and in which, finally, R' and R'' can together form a butylene or pentylene radical.

4. Process according to one of the preceding claims for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used an aminosilane $R_3Si\ NR'R''$, the various symbols having one of the meanings given above.

5. Process according to one of Claims 1 to 3 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a diaminosilane $R_2Si(NR'R'')_2$, the various symbols having one of the meanings given above.

6. Process according to one of Claims 1 to 3 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a silylurea

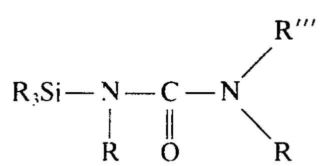

the various symbols having one of the meanings given above.

7. Process according to one of Claims 1 to 3 for obtaining monomeric or polymeric compounds having isocyanurate groups by catalytic cyclotrimerisation of isocyanates in which the isocyanate group is not directly bonded to a carbon atom belonging to an aromatic group, characterised in that as the compound initiating the catalytic reaction there is used a silazane

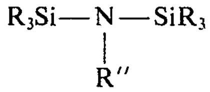

the various symbols having one of the meanings given above.

8. Process according to one of Claims 1 to 7 in which a polyisocyanate-polyisocyanurate is obtained by catalytic cyclotrimerisation of an aliphatic or cycloaliphatic polyisocyanate or an adduct or prepolymer and in which the catalyst is destroyed when the desired isocyanurate content has been reached.

9. Process according to Claim 8 in which the catalyst is destroyed by adding an acid compound.

10. Process for obtaining a polyisocyanate-polyisocyanurate by cyclotrimerisation of an aliphatic or cycloaliphatic diisocyanate according to one of Claims 8 and 9, characterised in that at the end of the reaction the residual monomeric diisocyanate is removed.

11. Process according to one of Claims 1 to 10 in which the polyisocyanate is hexamethylene diisocyanate.